(19) Europäisches Patentamt / European Patent Office / Office européen des brevets

(11) **EP 3 981 383 A1**

(12) **EUROPEAN PATENT APPLICATION**

(43) Date of publication:
13.04.2022 Bulletin 2022/15

(21) Application number: 21210194.3

(22) Date of filing: 08.11.2017

(51) International Patent Classification (IPC):
*A61K 8/27* (2006.01)  *A61K 8/36* (2006.01)
*A61Q 15/00* (2006.01)

(52) Cooperative Patent Classification (CPC):
**A61K 8/27; A61K 8/361; A61Q 15/00**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: 10.11.2016 GB 201619015

(62) Document number(s) of the earlier application(s) in
accordance with Art. 76 EPC:
**17794743.9 / 3 538 058**

(71) Applicant: **Givaudan SA
1214 Vernier (CH)**

(72) Inventor: **BROOKS, Matthew Peter
Ashford, TN24 0BS (GB)**

(74) Representative: **Global Patents
Givaudan SA
Grafenaustrasse 7
6300 Zug (CH)**

Remarks:
This application was filed on 24.11.2021 as a
divisional application to the application mentioned
under INID code 62.

(54) **DEODORIZING AGENT COMPRISING ZINC NEODECANOATE**

(57) A scented deodorizing agent comprising zinc neodecanoate, which is useful to provide a deodorancy benefit and a sustained scent benefit.

EP 3 981 383 A1

## Description

Field of the Invention

This disclosure is concerned with deodorizing agents useful in preventing or suppressing human, animal and environmental malodour, and in particular human body malodour, as well as deodorant compositions containing same.

Background of the Invention

Deodorant active agents are ingredients, or formulations, that are designed either to mask malodours once they are formed, or to prevent or hinder their formation.

The former method (masking) involves the use of fragrance ingredients, acting in the gaseous phase to create an olfactory signal that reduces a human subject's perception of any malodour present.

The latter method (malodour prevention or suppression) employs deodorant active agents to eliminate malodorous ingredients either directly reacting or interacting with them chemically, or by deactivating or interrupting the pathways by which malodorous ingredients are formed. Taking human body malodour as an example, the latter method either involves the use of bactericides or bacteriostatic agents to disrupt local populations of micro-organisms that act on malodour precursor compounds contained in sweat to generate malodours, and/or it involves the use of ingredients, such as astringent anti-perspirant ingredients (typically aluminium or zirconium salts) to prevent or reduce the flux of sweat.

The principal disadvantage of many deodorant or antiperspirant active ingredients is that some of the commonly employed ingredients, such as the aforementioned aluminium or zirconium salts or bactericides and bacteriostatic agents, are perceived as being unfriendly to human skin, or to populations of micro-flora contained on human skin. Consequently, there is a desire in the industry to reduce or even eliminate the use of these materials. This is particularly the case with regard to their use in cosmetic products intended to be applied topically on human skin.

Zinc carboxylates represent a genus of deodorant active ingredients, generally recognized as safe, that act to exert a malodour-counteracting or eliminating effect by interacting or reacting chemically with malodorous ingredients, such as the myriad sulphur- or nitrogen-containing ingredients, or short-chain fatty acids, found in sweat. Zinc ricinoleate is a particular example of a zinc carboxylate that is often mentioned in the prior art in the context of deodorant applications.

Substantially all deodorant compositions will combine both odour-masking ingredients (fragrances) and odour-preventing/suppressing active ingredients. A challenge for formulators, therefore, is to ensure that the odour-preventing/suppressing active ingredients and the odour masking ingredients do not interfere with each other, but rather complement each other in order that the overall odour-prevention/suppression/masking attributes of these ingredients, as well as the hedonic effects, are exploited to the full.

For example, whilst fragrance ingredients can play an important odour-masking role in deodorant compositions, they are also employed in deodorant compositions with aesthetic considerations in mind, and the use of a deodorant active ingredient should not cause or contribute to deodorant compositions having an unbalanced, unappealing or even unpleasant scent impression.

On the other hand, deodorant active ingredients, such as the zinc carboxylates, that interact chemically to absorb malodorous compounds are generally regarded to lack efficacy unless their incorporation levels in deodorant compositions are sufficiently high. In order to exert an acceptable odour-preventing/suppressing effect, these active ingredients need to be easily incorporated into fragrance-containing deodorant composition bases, and preferably be readily soluble or miscible therein at efficacious levels.

However, it is a particular disadvantage with the malodour-counteracting zinc carboxylates that they are not easily incorporated into fragrance-containing deodorant compositions. In particular, residual free acids contained in lower molecular weight homologue zinc carboxylates tend to be foul-smelling and can disturb and unbalance the scent impression of any deodorant composition containing them. On the other hand, higher molecular weight homologue zinc carboxylates, such as zinc ricinoleate, owing to the relatively low levels of zinc ions for a given mass of zinc carboxylate and are consequently difficult to load at sufficiently high levels in deodorant bases to impart an acceptable deodorancy effect. Their lack of efficacy can be further exacerbated by their poor solubility in deodorant bases. Furthermore, poor solubility impact on their deodorizing efficacy, the insoluble material when administered to a site in need of treatment, can leave unsightly residues. Still further, when deodorant compositions are in aerosol format, un-dissolved material can cause nozzle clogging.

There remains a need to provide deodorant active ingredients and deodorant compositions containing same that can deliver high levels of perceived malodour-reduction when applied to a site containing a malodorous source. There is a particular need for deodorant active ingredients and deodorant compositions containing same that can deliver high levels of perceived malodour-reduction when applied topically to a human subject, and particularly the under arm area of a human subject. There is, moreover, a need to provide deodorant actives that can be easily incorporated at efficacious levels into fragrance-containing deodorant compositions, and which do not cause or contribute to an unbalanced, unappealing or even an unpleasant overall scent impression.

Summary of the Invention

We have discovered that the performance of deodorant compositions, and in particular fragrance-containing deodorant compositions useful in the control of human, animal and environmental malodours, but particularly human body odour, can be improved by the addition to said composition of a deodorizing agent containing zinc neodecanoate. More particularly, we found that zinc neodecanoate can be incorporated into deodorant composition bases in a surprisingly facile manner. Furthermore, such deodorant compositions applied to a site of a source of malodour can deliver a deodorancy benefit and a sustained, undistorted and pleasant scent impression.

Accordingly, the present invention is directed in a first aspect to a deodorizing agent comprising zinc neodecanoate.

In another aspect the invention is directed to a deodorant composition, in particular a fragrance-containing deodorant composition, comprising the deodorizing agent of the present invention.

In an embodiment of the invention, said deodorant composition is an antiperspirant deodorant composition.

In an embodiment of the invention, said deodorant composition is an antiperspirant deodorant composition that is free or substantially free of aluminium and/or zirconium astringent antiperspirant agents.

In an embodiment of the invention, said deodorant composition is an anhydrous deodorant composition.

The present invention also provides a method of delivering a deodorancy benefit to a site of a source of malodour, said method comprising the step of applying to the site, or to a location proximate to the site, a deodorant composition of the present invention in order to prevent, reduce or suppress emanation of a malodour into a space proximate to said source.

The invention further provides a method of providing a deodorancy benefit and a sustained scent benefit to a site of a source of malodour, said method comprising the step of applying to the site, or to a location proximate to the site, a fragrance-containing deodorant composition of the present invention.

In an embodiment of the invention, there is provided a method of delivering a deodorancy benefit to a site of a source of malodour, said method comprising the step of applying to the surface of a human body the deodorant composition of the present invention.

In an embodiment of the invention, there is provided a method of delivering a deodorancy benefit and a sustained scent benefit to a site of a source of malodour, said method comprising the step of applying to the surface of a human body the deodorant composition of the present invention.

In a related aspect, the invention is directed to a method of manufacturing a deodorant composition according to the present invention, said method comprising the step of incorporating a deodorizing agent comprising zinc neodecanoate into a deodorant base, in which the deodorizing agent is soluble or miscible.

The invention further relates to the use of zinc neodecanoate as a deodorizing agent. In particular, zinc neodecanoate may be used as a deodorizing agent for preventing or suppressing human, animal and environmental malodour, and especially human body malodour.

These and other aspects and embodiments of the invention will be explained in further detail with reference to the following detailed description and examples.

Detailed Description

The deodorizing agent according to the present invention should be in the form of a pourable liquid at ambient temperature or at the temperature at which it is incorporated into a deodorant composition. Zinc neodecanoate in neat form is a pourable liquid and can be used as the deodorizing agent as such.

Accordingly, in an embodiment of the present invention, the deodorizing agent consists essentially of zinc neodecanoate in neat form.

In another embodiment of the invention, the deodorizing agent may be in the form of a solution or a blend consisting essentially of zinc neodecanoate and a solvent therefor compatible with deodorant applications. For example, those solvents having a clog P of 1.5 or greater can be generally used.

The deodorizing agent of the present invention may further comprise a fragrance component comprising one or more fragrance ingredients. Therefore, in an embodiment of the invention, the deodorizing agent consists essentially of zinc neodecanoate dissolved in or miscible with a fragrance component comprising at least one fragrance ingredient.

Alternatively or in addition, the deodorizing agent may also comprise a solvent, such as isopropyl myristate.

The levels of zinc neodecanoate in the deodorizing agent can be from about 1 wt % to about 100 wt % based on the total weight of the deodorizing agent.

Zinc neodecanoate is commercially available and can be prepared according to well-known procedures. Preferably, zinc neodecanoate is prepared from purified neodecanoic acid.

Suitable methods for purifying neodecanoic acid are described in examples 5 and 6 below, for instance.

In a particular embodiment of the invention, the deodorizing agent is a fragrance-containing deodorizing agent comprising zinc neodecanoate dissolved in or miscible with a fragrance component comprising at least one fragrance ingredient.

Any fragrance ingredients may be employed in the fragrance component. Non-limiting examples of fragrance ingredients suitable for use are described in standard reference works used in the perfumery industry, for example in Arctander, Perfume and Flavour Chemicals (Aroma Chemicals), vol. I and II (1969), and any later volumes and editions of that work.

In a particular embodiment of the invention, the deodorizing agent may contain at least one fragrance ingredient that is particularly efficient at exerting a malodour counteracting effect. Malodour-counteracting fragrance ingredients are known in the art. However, we have found certain fragrance ingredients that are particularly effective malodour-counteracting ingredients when used alone or in combination. These ingredients include, but are not limited to, the following GROUP A and GROUP B ingredients:

GROUP A ingredients are selected from the group consisting of 3,7-dimethyloct-6-enal (e.g. Citronellal); , 3,7-dimethyloct-6-en-1-ol (e.g. Citronellol); 2,4-dimethylcyclohex-3-enecarbaldehyde (e.g. Cyclal C); (E)-dec-4-enal; ethyl 2-methyl butyrate; 1-phenylethyl acetate (e.g. Gardenol); (Z)-hex-3-en-1-yl acetate; hexyl acetate; isoamyl acetate; Litsea cubeba oil; nonanal; Orange oil; Orange terpenes; prenyl acetate; 4-methyl-2-(2-methylprop-1-en-1-yl)tetrahydro-2H-pyran (e.g. Rose Oxide); 4-methylene-2-phenyltetra-hydro-2H-pyran (e.g. Rosyrane super); 2,4-dimethylcyclohex-3-enecarbaldehyde (e.g. Tricyclal);

GROUP B ingredients are selected from the group consisting of 2,6,10-trimethylundec-9-enal (e.g. Adoxal); Armoise oil Morocco; 8-(sec-butyl)-5,6,7,8-tetrahydroquinoline (e.g. Bigaryl); (2E)-3-phenyl-prop-2-enal (e.g. Cinnamic aldehyde); (E)-3,7-dimethylocta-2,6-dienal (e.g. Citral); ethyl caproate; 1,3,3-trimethyl-2-oxabicyclo[2.2.2]octane (e.g. Eucalyptol); Eucalyptus oil (e.g. Eucalyptus globulus oil China); 1-(1,2,8,8-tetramethyl-1,2,3,4,5,6,7,8-octahydronaphthalen-2-yl)ethanone (e.g. Georgywood); hexyl isobutyrate; (E)-4-(2,6,6-trimethylcyclohex-1-en-1-yl)but-3-en-2-one (e.g. Ionone beta); isobutyl isobutyrate; isobutyl quinolone; isopropyl methyl-2-butyrate; (2E,6Z)-3,7-dimethylnona-2,6-dienenitrile (e.g. Lemonile); 3,7-dimethylocta-1,6-dien-3-ol (e.g. Linalool); 2,6-dimethylhept-5-enal (e.g. Melonal); methyl amyl ketone; methyl benzoate; methyl heptenone; methyl hexyl ketone; phenyl ethyl acetate; tetrahydro myrcenol; Patchouli Oil; tridecen-2-nitrile; 6-methoxy-2,6-dimethyloctanal (e.g. Calypsone); 5-tert-butyl-2-methyl-5-propyl-2H-furan (e.g. Cassyrane); (4E)-9-hydroxy-5,9-dimethyl-4-decenal (e.g. Mahonial); 1-methyl-2-(5-methylhex-4-en-2-yl)cyclopropyl)methanol (e.g. Rosyfolia); and 3-(4-isobutyl-2-methylphenyl)propanal (e.g. Nympheal).

In a more particular embodiment, the fragrance component may be provided such that it contains at least 2, at least 3, at least 4, or at least 5 of the following perfume GROUP A ingredients:

3,7-dimethyloct-6-enal (e.g. Citronellal); 3,7-dimethyloct-6-en-1-ol (e.g. Citronellol); 2,4-dimethylcyclohex-3-enecarbaldehyde (e.g. Cyclal C); (E)-dec-4-enal; ethyl 2-methyl butyrate; 1-phenylethyl acetate (e.g. Gardenol); (Z)-hex-3-en-1-yl acetate; hexyl acetate; isoamyl acetate; Litsea cubeba oil; nonanal; Orange oil; Orange terpenes; prenyl acetate; 4-methyl-2-(2-methylprop-1-en-1-yl)tetrahydro-2H-pyran (e.g. Rose Oxide); 4-methylene-2-phenyltetrahydro-2H-pyran (e.g. Rosyrane super); 2,4-dimethylcyclohex-3-enecarbaldehyde (e.g. Tricyclal);

and optionally at least 2, at least 3, at least 4, or at least 5 of the following GROUP B ingredients: 2,6,10-trimethylundec-9-enal (e.g. Adoxal); Armoise oil Morocco; 8-(sec-butyl)-5,6,7,8-tetrahydroquinoline (e.g. Bigaryl); (2E)-3-phenylprop-2-enal (e.g. cinnamic aldehyde); (E)-3,7-dimethylocta-2,6-dienal (e.g. Citral); ethyl caproate; 1,3,3-trimethyl-2-oxabicyclo[2.2.2]octane (e.g. Eucalyptol); Eucalyptus oil (e.g. Eucalyptus globulus oil China); 1-(1,2,8,8-tetramethyl-1,2,3,4,5,6,7,8-octahydronaphthalen-2-yl)ethanone (e.g. Georgywood); hexyl isobutyrate; (E)-4-(2,6,6-trimethylcyclohex-1-en-1-yl)but-3-en-2-one (e.g. Ionone beta); ilsobutyl isobutyrate; isobutyl quinolone; isopropyl methyl-2-butyrate; (2E,6Z)-3,7-dimethylnona-2,6-dienenitrile (e.g. Lemonile); 3,7-dimethylocta-1,6-dien-3-ol (e.g. Linalool); 2,6-dimethylhept-5-enal (e.g. Melonal); methyl amyl ketone; methyl benzoate; methyl heptenone; methyl hexyl ketone; phenyl ethyl acetate; tetrahydro myrcenol; Patchouli Oil; tridecen-2-nitrile; 6-methoxy-2,6-dimethyloctanal (e.g. Calypsone); 5-tert-butyl-2-methyl-5-propyl-2H-furan (e.g. Cassyrane); (4E)-9-hydroxy-5,9-dimethyl-4-decenal (e.g. Mahonial); 1-methyl-2-(5-methylhex-4-en-2-yl)cyclopropyl) methanol (e.g. Rosyfolia); and 3-(4-isobutyl-2-methylphenyl)propanal (e.g. Nympheal).

Preferred fragrance ingredients include, for instance, ambrofix (dodecahydro-3a,6,6,9a-tetramethyl naphtho[2,1-b]furan), radjanol (2-ethyl-4-(2,2,3-trimethyl-cyclopent-3-enyl)-but-2-en-1-ol), benzyl salicylate, cis-3-hexenyl salicylate, coumarin, cyclamen aldehyde, dihydrojasmonate, hedione (methyl dihydrojasmonate), lilial (3-(4-tert-butylphenyl)-2-methylpropanal), nympheal (3-(4-isobutyl-2-methylphenyl)propanal), methyl ionone, phenyl ethyl phenyl acetate, tetrahydrogeranyl acetate, trimofix (reaction products of acetic anhydride and 1,5,10-trimethyl-1,5,9-cyclodecatriene), and tropional (3-(1,3-benzodioxol-5-yl)-2-methylpropanal).

The fragrance component, or any fragrance ingredients forming a part of the fragrance component may be presented in the form of free fragrance oil, or in encapsulated form, or both as free oil and in encapsulate form. Suitable encapsulated media include matrices of water soluble, modified starch.

Starches suitable for encapsulating fragrances are modified starches, which can be made from, raw starch, pre-gelatinized starch, modified starch derived from tubers, legumes, cereal and grains, for example corn starch, wheat starch, rice starch, waxy corn starch, oat starch, cassava starch, waxy barley, waxy rice starch, sweet rice starch, amioca, potato starch, tapioca starch and mixtures thereof.

Modified starches suitable for use as the encapsulating matrix include starches that are modified chemically, physically, e.g. through heat or pressure, or enzymatically. They include hydrolyzed starch, acid thinned starch, starch esters of long chain hydrocarbons, starch acetates, starch octenyl succinate, and mixtures thereof. Starch esters having a degree of substitution in the range of from about 0.01% to about 10.0% may be used to encapsulate the fragrance ingredients. The hydrocarbon part of the modifying ester should be from a C5 to C16 carbon chain.

The deodorizing agent is readily incorporated into all manner of deodorant composition bases, in any product format. In particular, the deodorizing agent is dissolvable or miscible in deodorant composition bases at levels that provide efficacious deodorancy effects. In particular, a deodorizing agent can be incorporated into a deodorant composition base at any level necessary to achieve a desired deodorizing effect.

Particular deodorant compositions of the invention may contain levels of deodorizing agent such that the finished deodorant composition comprises up to about 20 wt % of zinc neodecanoate, more particular 2 to 10 wt %, more particularly still about 2 to 5 wt %, e.g. about 4 wt %. Given the fact that the zinc neodecanoate is dissolved in or is miscible with a deodorant composition, it does not leave any unsightly residues when it is applied to a site in need of treatment, or to any other surfaces that come into contact with the treated site.

Furthermore, we found that zinc neodecanoate applied to a surface in need to treatment, for example the human skin, forms a thin film, which is somewhat occlusive and allows the fragrance component to evaporate slowly over time. This results in a substantially linear release of fragrance over a sustained period of time. This surprising finding allows the preparation of deodorant compositions to deliver substantially constant scent impression over a sustained period of time.

The deodorant compositions of the present invention may be used to eliminate malodours as the form, e.g. in the axilla. Key odorants in axillary sweat include: short chain fatty acids, e.g. 3-hydroxy-3-methyl hexanoic acid (HMHA), 3-methyl-2-hexenoic acid (Schizophrenic acid), or 4-ethyl octanoic acid (Goat acid); sulphanylalcohols (thiols) with low olfactory thresholds, e.g. 3-methyl-3-sulphanylhexan-1-ol (sweat thiol), 2-methyl-3-sulphanylbutan-1-ol (onion thiol), or 3-mercapto-hexan-1-ol; steroids, e.g. $5\alpha$-androst-16-en-3-one or $5\alpha$-androst-16-en-$3\alpha$-ol. Other acids are also often present, e.g. butyric acid, isovaleric acid, hexanoic acid, or octanoic acid. In particular, the deodorant compositions of the present invention can scavenge thiols as they emanate in the axilla.

The deodorizing agent of the present invention can be used in any deodorant composition product formats known in the art for application to sites containing, or proximate to sites containing, sources of human, animal or environmental malodour. The deodorizing agent is particularly useful in the topical application to the human body, and more particularly still, the human axilla. Suitable product formats include wax-based sticks, soap-based sticks, roll-on suspensions, solutions, emulsions or gels, squeeze-sprays, pump-sprays and aerosols.

In addition to deodorizing agent of the present invention, each deodorant composition product format can contain its own selection of additional components conventionally employed in the particular product format. The types of additional components are well known in the art, and a non-limiting selection of such components is listed herein below.

Deodorant compositions of the present invention include a fragrance component and an emollient system, and may contain any number of optional ingredients that are described below and/or known to the skilled artisan.

The fragrance component may be incorporated into the deodorant compositions as part of the deodorizing agent, as described herein above, or as a part of the deodorant composition base.

In an alternative embodiment, the deodorizing agent may be in the form of a kit of parts comprising a first part consisting essentially of zinc neodecanoate, or a solution of zinc neodecanoate in a suitable solvent, and a second part comprising the fragrance component. In a further alternative, the fragrance component may be split between said first and second parts. The parts are intended to be incorporated separately, sequentially or simultaneously to a deodorant composition base during the preparation of a deodorant composition.

The levels of fragrance component contained in the deodorant compositions should be effective to provide a desired scent impression and/or to provide malodour prevention/suppression or masking.

Generally, the deodorant compositions of the present invention may comprise the total fragrance component at concentrations ranging from about 0.05 wt % to about 10 wt % based on the total amount of the deodorant composition.

Deodorant compositions of the present invention may contain an emollient composition that may consist of at least one or a combination of different emollient ingredients. The emollients are preferably liquid under ambient conditions, and comprise at least one emollient having a solubility parameter greater than about 9 and more preferably greater than about 11, and a vapour pressure below about 2 mm Hg at 25 °C. Depending on the type of product form desired, concentrations of the emollient(s) in the deodorant compositions can range from about 10 wt % to about 90 wt % based on the total weight of the deodorant composition.

Emollients suitable for use in the deodorant compositions of the present invention include, but are not limited to, topically safe and effective organic, silicone-containing or fluorine-containing, volatile or nonvolatile, polar or non-polar, provided that the resulting combinations of emollients form a solution or other homogenous liquid or liquid dispersion at the selected processing temperature of the composition. Processing temperatures for the deodorant compositions can range from about 15 °C to about 150 °C depending on product form. Preferred emollients with the requisite solubility parameter and volatility include but are not limited to: propylene glycol, dipropylene glycol, tripropylene glycol, diethylene glycol, triethylene glycol, PEG-4, PEG-8, 1,2 pentanediol, 1,2 hexanediol, hexylene glycol, and glycerin.

Other emollients can be included in the product to provide other benefits to the deodorant such as, for example, good skin feel or skin moisturization. Other examples of suitable emollients include C2 to C20 monohydric alcohols, C2 to C40 dihydric or polyhydric alcohols, alkyl ethers of polyhydric and monohydric alcohols, volatile silicone emollients such as cyclopentasiloxane, non-volatile silicone emollients such as dimethicone, mineral oils, polydecenes, and petrolatum.

Deodorant compositions may contain thickening or structuring agents in order to provide a desired hardness and application characteristics to the compositions. The thickening or structuring agent concentrations may range from about 0.1 wt % to about 30 wt %, based on the total weight of the deodorant composition depending on the type of product and thickening or structuring agent. Preferred inclusion ranges for stick products are from about 4 to about 20 wt %.

If the deodorant composition is aqueous-based, it may comprise a thickening or structuring agent that can melt to form a solution or other homogenous liquid or liquid dispersion within the liquid carrier at a processing temperature of from about 50 °C or to about 150 °C.

Suitable thickening or structuring agents for use in the aqueous and anhydrous deodorant compositions of the present invention include, but are not limited to, fatty acid gellants, salts of fatty acids, hydroxy fatty acid gellants, esters and amides of fatty acid or hydroxy fatty acid gellants, cholesterolic materials, dibenzylidene alditols, lanolinolic materials, fatty alcohols, triglycerides, and other suitable gellants. Other examples include finely divided or colloidal silicas, fumed silicas, and silicates, which includes montmorillonite clays and hydrophobically treated montmorillonites, e.g., bentonites, hectorites and colloidal magnesium silicates.

Preferred thickening or structuring agents for use in the aqueous and anhydrous deodorant compositions are the solid salts of fatty acids wherein the fatty acid moiety has from about 12, from about 16 or from about 18 carbon atoms to about 40, to about 22, or about 20 carbon atoms. Suitable salt forming cations for use with these thickening or structuring agents include metal salts such as alkali metals (e.g. sodium and potassium), alkaline earth metals (e.g. magnesium), and aluminum. Preferred are sodium and potassium salts. For example, suitable salt forming cations may be selected from the group consisting of sodium stearate, sodium palmitate, potassium stearate, potassium palmitate, sodium myristate, aluminum monostearate, and combinations thereof.

Depending on the chosen product form, the deodorant compositions of the present invention may employ a propellant. Suitable propellants include, but are not limited to, dimethylether, 1,1-difluoroethane, 1,1,1,2-tetrafluoro ethane, carbon dioxide, butane, isobutane, propane, isopentane, pentane, nitrous oxide, carbon dioxide, halogenated hydrocarbons such as trichlorofluoromethane, dichlorodifluoromethane, dichlorotetrafluoroethane trichlorotrifluoroethane, trichlorotetrafluoroethane, and monochlorodifluoromethane, and combinations thereof. The total propellant concentration in the deodorant compositions can range from about 5% to about 99%, more typically from about 15% to about 90%, even more preferably from about 20% to about 70%, by weight of the deodorant composition.

In addition to the aforementioned components, the deodorant compositions of the present invention may further comprise one or more components, which may modify the physical or chemical characteristics of the compositions. Non-limiting examples of such components include, but are not limited to, pH buffering agents, additional malodour controlling agents, humectants, soothing agents, dyes and pigments, medicaments, baking soda and related materials, preservatives, and soothing agents such as aloe vera, allantoin, D-panthenol, avocado oil and other vegetative oils, and lichen extract.

Suitable additional deodorant active ingredients may include any topical material that is known or otherwise effective in preventing or eliminating malodour associated with perspiration. Suitable deodorant active ingredients may be selected from the group consisting of antimicrobial agents (e.g., bactericides, fungicides), malodour-absorbing material, and combinations thereof. For example, antimicrobial agents may comprise cetyltrimethylammonium bromide, cetyl pyridinium chloride, benzethonium chloride, diisobutyl phenoxy ethoxy ethyl dimethyl benzyl ammonium chloride, sodium N-lauryl sarcosine, sodium N-palmethyl sarcosine, lauroyl sarcosine, N-myristoyl glycine, potassium N-lauryl sarcosine, trimethyl ammonium chloride, sodium aluminum chlorohydroxy lactate, triethyl citrate, tricetylmethyl ammonium chloride, 2,4,4'-trichloro-2'-hydroxy diphenyl ether (triclosan), 3,4,4'-trichlorocarbanilide (triclocarban), diaminoalkyl amides such as L-lysine hexadecyl amide, heavy metal salts of citrate, salicylate, and piroctose, especially zinc salts, and acids thereof, heavy metal salts of pyrithione, especially zinc pyrithione, zinc phenolsulfate, farnesol, and combinations thereof.

The deodorant compositions according to the present invention may be anti-perspirant deodorant compositions. In such cases, they will contain antiperspirant active agents.

Antiperspirant active ingredients suitable for use in the antiperspirant/deodorant compositions of the present teachings can include any compound, composition or other material having antiperspirant activity. Active antiperspirant ingredients include astringent metallic salts, especially the inorganic salts of aluminum, zirconium, and zinc, as well as their mixtures. Depending on the product form and intended use, aluminum and zirconium salts may be used, such as aluminum halides, aluminum chlorohydrate, aluminum hydroxyhalides, zirconyl oxyhalides, zirconyl hydroxyhalides, and mixtures thereof. Additional antiperspirant ingredients that may be included in the formation include: aluminum chloride, aluminum chlorohydrate, aluminum chlorohydrex polyethylene glycol, aluminum chlorohydrex propylene glycol, aluminum dichlorohydrate, aluminum dichlorohydrex polyethylene glycol, aluminum dichlorohydrex propylene glycol, aluminum sesquichlorohydrate, aluminum sesquichlorohydrex polyethylene glycol, aluminum sesquichlorohydrex propylene glycol, and aluminum zirconium octachlorohydrate.

Preferably, however, deodorant compositions of the present invention are free, or substantially free of aluminium- or zirconium-based antiperspirant active ingredients.

In one embodiment, the deodorizing agent and deodorant compositions of the present invention are free or substantially free of thiol compounds.

In one embodiment, the deodorizing agent and deodorant compositions of the present invention are free or substantially free of polar oil derivatives, in particular of polar oil derivatives having an Inorganic Organic Balance (I.O.B.) value of 0.1 or more.

The deodorant compositions of the present invention can be formulated in any of the known or otherwise effective product formats. Representative product formats include solid and solid-like forms (e.g., sticks, waxes, powders), liquids (e.g., aerosol sprays, pump sprays, mist sprays, roll-ons, wipes), and semi-solids (e.g., gels, creams, soft solids, lotions).

Aerosol compositions are a preferred product format according to the invention.

A propellant is a major component of an aerosol composition and the remainder of the deodorant composition may contain any of the ingredients referred to hereinabove, useful or conventionally used in such product formats.

Particularly preferred product formats are anhydrous aerosol products.

An anhydrous deodorant composition is a product that contains less than 2 wt %, more particularly less than 0.5 wt % water, and especially free of water. More particularly, any water that is present in the product is not added during the preparation of the product, but is merely present as a residue that may be contributed by any of the mixed components.

In another aspect, the invention is directed to a method of manufacturing a deodorant composition referred to herein above, said method comprising the step of incorporating the deodorizing agent into a deodorant base, in which the deodorizing agent is soluble or miscible.

For purposes of maintaining or enhancing deodorant efficacy or retaining a pleasant scent impression, it may be beneficial to separate one or more fragrance ingredients and the deodorizing agent to prevent their premature mixing or even to maintain their compatibility. The separation can be effected by maintaining these components in physically separate locations. One or more fragrance ingredients can be encapsulated to maintain separation from zinc neodecanoate.

The deodorant compositions according to the present invention can be prepared by any effective technique, suitable for providing a deodorant composition of the desired form and having the ingredients described herein. Many such techniques are described in the deodorant composition arts for the described product forms.

A deodorant composition in aerosol format is filled into canisters capable of withstanding pressures generated by the composition, employing conventional filling apparatus and conditions. The canister can conventionally be a commercially available metal canister fitted with a dip tube, valve and spray nozzle through which the formulation is dispensed.

Deodorant compositions are useful for all manner of applications concerned with the treatment of human, animal or environment malodour, including uses in home care, oral care, personal care and fabric care products.

Home care includes air fresheners, dishwashing product, surface cleaners, toilet cleaners, and absorbent products, such as diapers and feminine hygiene products.

Oral care includes tooth pastes and mouthwashes.

Personal care includes hair care, deodorant or antiperspirant deodorant products, bath and shower products and soaps.

Fabric care includes pre-treatment products, detergents and conditioners or softeners.

Deodorant compositions of the present invention may be applied to a site containing a source of malodour in any known or otherwise effective method. Such methods include the application to a site of a safe and efficacious amount of the deodorant composition.

The deodorant compositions of the present invention are particularly useful in the treatment of human body odours by the topical application of the composition to the axilla or other area of the skin in any known or otherwise effective method for controlling malodor associated with perspiration. These methods comprise applying to the axilla or other area of the human skin a safe and effective amount of the deodorant composition of the present invention.

Thus, the present invention also refers to the use of zinc neodecanoate as a deodorizing agent, or in a deodorizing agent. This is particularly useful for the applications described above.

There now follows a series of examples that serve to illustrate the invention.

Example 1: Sensory test comparing zinc neodecanoate and zinc ricinoleate

Aerosols were prepared using the following two test formulations, respectively:

- 4% zinc neodecanoate, 12% cyclomethicone, 84% propellant

- 4% zinc ricinoleate, 12% cyclomethicone, 84% propellant

(propellant = isobutane/propane mix)

5 days prior to testing, the male subjects substituted their normal body wash with an unperfumed product (Sanex 0% Bodywash) and were asked to only use a specific deodorant supplied (Lynx Attract for Him).

On the morning before the test, the male subjects were asked to shower using an unperfumed shower gel provided and not use any antiperspirant product or apply any other fragranced products.

Subjects reported to a panel suite in the morning in two groups for test product application.

The administrator applied 1.0 g of each product to each subject's axilla. Each subject wore both of the test formulations (one on each axilla region). The manner in which each test formulation was applied (left or right axilla) was randomised. Each of a subject's axilla was then coded with a random code number.

The subjects were asked not to shower until after the test had been completed the next day. The subjects were also provided with a list of rules, by which they had to abide for the duration of the test. The rules included no swimming and no gym activity between test formulation application and assessment.

The subjects' axillae were thereafter tested by sensory panellists. The members of the sensory panels had been selected on a basis of their olfactory sensory acuity and had thereafter received training for a period of 4 - 6 months. Their training enabled them to identify individual odour characteristics and score their perceived intensity against given standards in a consistent manner.

The sensory panellists were asked to rate the perceived malodour intensity for each of the subject's axilla using a 0-100 line scale. The order of samples assessed was pre-determined using a balanced randomisation. The axillae were assessed in a sequential monadic way.

The panellists were asked to assess directly from the subject's axillae 24 hours after application. 10 subjects participated in the test.

The subjects were separated into two groups to be assessed by the panellists and each group was assessed by 22 panellists.

The data was analysed using an analysis of variance and multiple comparison test with a confidence level of 95%. It was found that the underarms treated with zinc neodecanoate had significantly lower malodour intensity than the underarms treated with zinc ricinoleate.

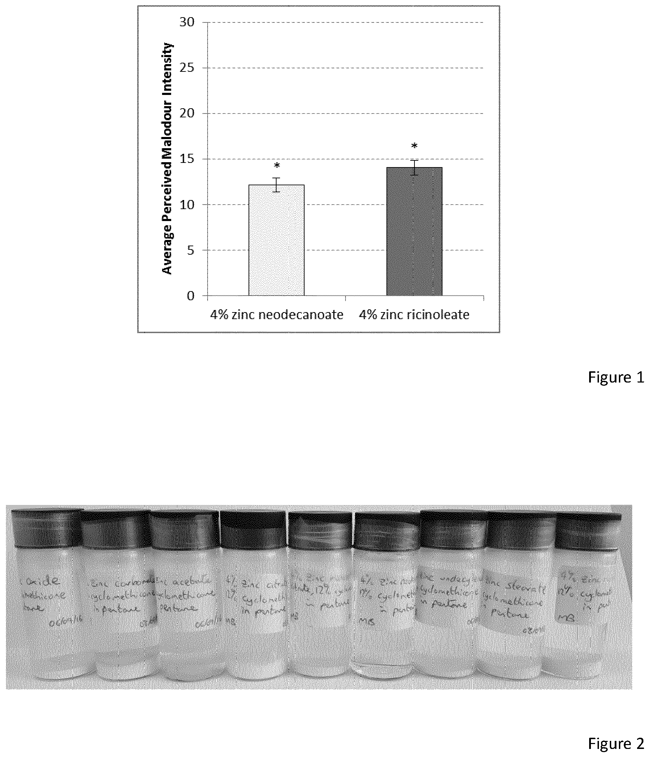

Results are shown graphically in Figure 1: The perceived malodour intensity for zinc neodecanoate was 12.1 (+/-0.8), whereas that for zinc ricinoleate was 14.0 (+/-0.8).

Example 2: Solubility Test: Comparison of zinc neodecanoate and zinc ricinoleate

The solubility of several zinc salts was explored using the following model aerosol base formulation:

- 4% zinc salt
- 12% cyclomethicone
- 84% pentane

(Pentane was used as a surrogate for isobutane/propane mix as it is easier to handle in a laboratory.)

Each salt and aerosol formulation was placed in a sealed glass vial that was agitated for several hours at room temperature. The resulting suspensions/solutions were allowed to stand overnight and any remaining solid matter present was noted.

It was found that the sample containing zinc neodecanoate was the only sample without any solid present.

The results are shown in Figure 2, which demonstrates the unique solubility of zinc neodecanoate compared with other zinc carboxylates. From left to right: zinc oxide, zinc carbonate, zinc acetate, zinc citrate, zinc mono octyl citrate, zinc neodecanoate, zinc undecylenate, zinc stearate, zinc ricinoleate.

Example 3: Fragrance linearity comparison of zinc neodecanoate and aluminium chlorohydrate

Aerosol compositions were prepared using the following two test formulations, respectively:

- 4% zinc neodecanoate, 1% fragrance, 12% cyclomethicone, 83% propellant

- 10% aluminium chlorohydrate, 0.8% disteardimonium hectorite, 1% fragrance, 12% cyclomethicone, 76.2% propellant

**(propellant = isobutane/propane mix)**

The aerosol compositions were sprayed (1.0 g) onto an aerosol pad, which was then laid out flat on a bench in a well-ventilated room. The process was repeated after 24 hours on new pads. Thus, some of the samples contained compositions that had been applied 24 hours before assessment, and on the others, the compositions had been applied only shortly before assessment. Each of the samples was labelled with a random number and assessed by a trained sensory panel.

The panellists were asked to rate the perceived fragrance intensity for each of the aerosol-containing pads using a 0-100 line scale. The order of samples assessed by the panellists was pre-determined using a balanced randomisation.

The data were analysed using an analysis of variance and multiple comparison test with a confidence level of 95%. It was found that there was no significant difference in fragrance intensity between the freshly applied samples. However, for the samples assessed after 24 hours, a significantly higher fragrance intensity was found for those with zinc neodecanoate.

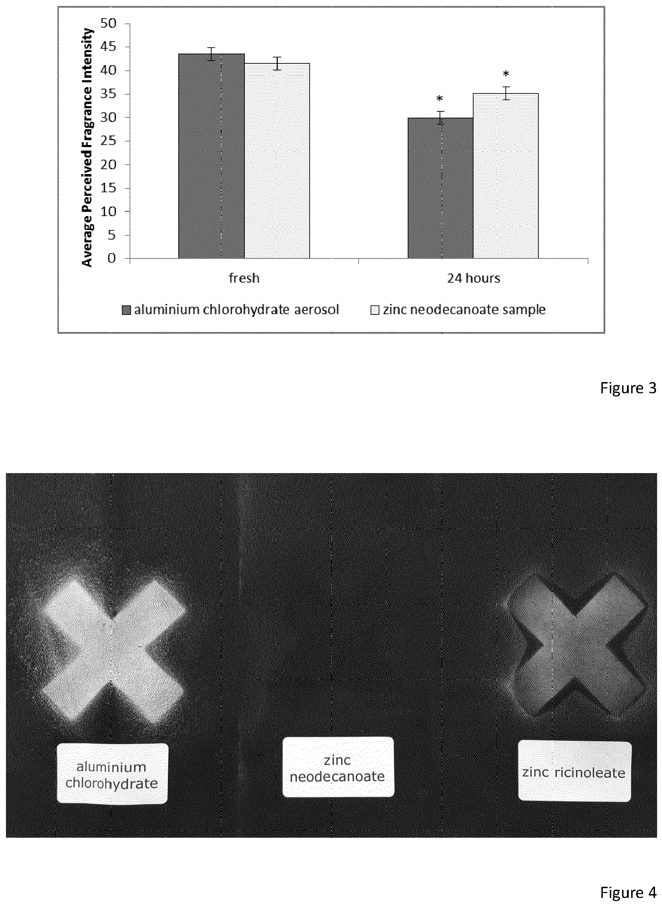

The results demonstrating the sustained scent impression of the zinc neodecanoate-containing composition are shown graphically in Figure 3.

Example 4: White marks

Aerosols were prepared using the following three test formulations, respectively:

- 4% zinc neodecanoate, 12% cyclomethicone, 84% propellant

- 4% zinc ricinoleate, 12% cyclomethicone, 84% propellant

- 10% aluminium chlorohydrate, 0.8% disteardimonium hectorite, 12% cyclomethicone, 76.2% propellant

**(propellant = isobutane/propane mix)**

Several card templates were prepared where a large X-shaped hole was present in each template. Each template was then placed over a black piece of cloth. The aerosols were shaken well, and then approximately 3 g of one of the three aerosols was dispensed over each template hole. Several minutes were allowed for any propellant to evaporate and the presence of any visible solid residues was noted.

It was found that only the aerosol containing zinc neodecanoate did not leave a powdered residue on the black cloth. The results are shown in Figure 4.

Example 5: Purification of neodecanoic acid by pressure hydrogenation and distillation

An autoclave vessel (Premex 120 mL) was charged with neodecanoic acid (70.0 g; Umecore) and Rhodium on alumina (1.0 g; Fluka puriss.) and was then pressurized with 100 bar hydrogen. The mixture was stirred at 150 °C for 32 h.

After pressure release and venting, the mixture was diluted with MTBE (100 mL) and filtered by suction. The filtrate was concentrated in a rotary evaporator under reduced pressure to yield 56.0 g (80% yield) of a colourless liquid, of which 54.6 g were distilled over a 15 cm Vigreux column at 80-85 °C (p = 0.12 mbar, bath temperature 128 °C).

Of the 8 collected fractions, N° 3-7 were olfactorily pure and combined to yield 32.3 g (46%) of a clear colourless oil. The material still had an intrinsic odour of its own, but lacked the sulfury/rubbery/phenolic off-notes unpurified neodecanoic acid typically has.

Example 6: Purification of neodecanoic acid by distillation

Neodecanoic acid (152.3 g; Umecore) was distilled over a 15 cm Vigreux column at 89-90 °C (p = 0.02 mbar, bath temperature 140-156 °C).

Of the 14 collected fractions, N° 8-14 were olfactorily pure and combined to yield 85.5 g (56%) clear colourless oil. Olfactory quality seemed similar to that of example 5.

**Claims**

1. A deodorizing agent useful in preventing or suppressing human body malodour, said deodorizing agent comprising zinc neodecanoate.

2. The deodorizing agent according to claim 1, wherein the zinc neodecanoate is prepared from purified neodecanoic acid.

3. The deodorizing agent according to claim 1 or claim 2, wherein the deodorizing agent consists essentially of zinc neodecanoate in neat form.

4. The deodorizing agent according to claim 1 or claim 2, in the form of a solution or a blend of zinc neodecanoate and a solvent therefor.

5. The deodorizing agent according to any one of claims 1 to 4, dissolved in or mixed with a fragrance component comprising at least one fragrance ingredient.

6. The deodorizing agent according to claim 4, wherein the fragrance component comprises at least one fragrance ingredient from GROUP A and/or at least one fragrance ingredient from GROUP B, wherein

GROUP A ingredients are selected from the group consisting of 3,7-dimethyloct-6-enal (e.g. Citronellal); 3,7-dimethyloct-6-en-1-ol (e.g. Citronellol); 2,4-dimethylcyclohex-3-enecarbaldehyde (e.g. Cyclal C); (*E*)-dec-4-enal; ethyl 2-methyl butyrate; 1-phenylethyl acetate (e.g. Gardenol); (*Z*)-hex-3-en-1-yl acetate; hexyl acetate; isoamyl acetate; Litsea cubeba oil; nonanal; Orange oil; Orange terpenes; prenyl acetate; 4-methyl-2-(2-methylprop-1-en-1-yl)tetrahydro-2H-pyran (e.g. Rose Oxide); 4-methylene-2-phenyltetrahydro-2H-pyran (e.g. Rosyrane super); 2,4-dimethylcyclohex-3-enecarbaldehyde (e.g. Tricyclal); and

GROUP B ingredients are selected from the group consisting of 2,6,10-trimethylundec-9-enal (e.g. Adoxal); Armoise oil Morocco; 8-(sec-butyl)-5,6,7,8-tetrahydroquinoline (e.g. Bigaryl); (2*E*)-3-phenylprop-2-enal (e.g. Cinnamic aldehyde); (*E*)-3,7-dimethylocta-2,6-dienal (e.g. Citral); ethyl caproate; 1,3,3-trimethyl-2-oxabicyclo[2.2.2]octane (e.g. Eucalyptol); Eucalyptus oil (e.g. Eucalyptus globulus oil China); 1-(1,2,8,8-tetramethyl-1,2,3,4,5,6,7,8-octahydronaphthalen-2-yl)ethanone (e.g. Georgywood); hexyl isobutyrate; (*E*)-4-(2,6,6-trimethylcyclohex-1-en-1-yl)but-3-en-2-one (e.g. Ionone beta); isobutyl isobutyrate; isobutyl quinolone; isopropyl methyl-2-butyrate; (2*E*,6*Z*)-3,7-dimethylnona-2,6-dienenitrile (e.g. Lemonile); 3,7-dimethylocta-1,6-dien-3-ol (e.g. Linalool); 2,6-dimethylhept-5-enal (e.g. Melonal); methyl amyl ketone; methyl benzoate; methyl heptenone; methyl hexyl ketone; phenyl ethyl acetate; tetrahydro myrcenol; Patchouli Oil; tridecen-2-nitrile; 6-methoxy-2,6-dimethyloctanal (e.g. Calypsone); 5-tert-butyl-2-methyl-5-propyl-2H-furan (e.g. Cassyrane); (4*E*)-9-hydroxy-5,9-dimethyl-4-decenal (e.g. Mahonial); 1-methyl-2-(5-methylhex-4-en-2-yl)cyclopropyl)methanol (e.g. Rosyfolia); and 3-(4-isobutyl-2-methylphenyl)propanal (e.g. Nympheal).

7. A deodorant composition comprising the deodorizing agent as defined in any one of claims 1 to 6, in particular an antiperspirant deodorant composition, more particularly an antiperspirant deodorant composition, which is free or substantially free of aluminium- and/or zirconium-based antiperspirant agents.

8. The deodorant composition according to claim 7, containing levels of deodorizing agent such that the finished deodorant composition comprises up to about 20 wt % of zinc neodecanoate, more particular 2 to 10 wt %, more particularly still about 2 to 5 wt %, e.g. about 4 wt %.

9. The deodorant composition according to claims 7 or 8, which is an anhydrous composition.

10. A method of delivering a deodorancy benefit to a site of a source of malodour, said method comprising the step of applying to the site, or to a location proximate to the site, a deodorant composition defined in any one of claims 7 to 9, in order to prevent, reduce or suppress emanation of a malodour into a space proximate to said source.

11. The method according to claim 10, providing a deodorancy benefit and a sustained scent benefit to a site of a source of malodour, said method comprising the step of applying to the site, or to a location proximate to the site, the scented deodorant composition defined in any one of claims 7 to 9.

12. The method according to claim 10 or claim 11, comprising the step of applying the deodorant composition to the surface of a human body.

13. A method of manufacturing the deodorant composition as defined in any one of claims 7 to 9, said method comprising the step of incorporating the deodorizing agent into a deodorant base, in which the deodorizing agent is soluble or miscible.

Figure 1

Figure 2

Figure 3

Figure 4

# EUROPEAN SEARCH REPORT

**Application Number**

EP 21 21 0194

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 145 707 A1 (SHISEIDO CO LTD [JP]) 17 October 2001 (2001-10-17) * claims 4, 14 * | 1-4,7, 10-13 | INV. A61K8/27 A61K8/36 A61Q15/00 |
| X | US 2009/176676 A1 (HILVERT JENNIFER ELAINE [US] ET AL) 9 July 2009 (2009-07-09) * paragraphs [0007] - [0012], [0081] * | 1-4,7, 10-13 | |
| X | US 2006/147390 A1 (SCHREIBER JORG [DE] ET AL) 6 July 2006 (2006-07-06) * claim 20 * | 1-4,7, 10-13 | |
| X | US 6 599 472 B1 (HUDSON ALICE P [US]) 29 July 2003 (2003-07-29) * example 18 * | 1-4,7, 10,11,13 | |
| X | JP H10 45543 A (TAIYO KORYO KK) 17 February 1998 (1998-02-17) * paragraphs [0004], [0007], [0008] * | 1-4,7,8, 10-13 | |
| A | | 5,6 | |
| A | US 2009/092568 A1 (MABROUK ISSA [US]) 9 April 2009 (2009-04-09) * paragraphs [0006], [0008], [0014], [0015] * | 1-13 | **TECHNICAL FIELDS SEARCHED (IPC)** A61K A61Q |

The present search report has been drawn up for all claims

| Place of search | Date of completion of the search | Examiner |
|---|---|---|
| Munich | 28 February 2022 | Simon, Frédéric |

EPO FORM 1503 03.82 (P04C01)

**EP 3 981 383 A1**

## ANNEX TO THE EUROPEAN SEARCH REPORT
## ON EUROPEAN PATENT APPLICATION NO.

EP 21 21 0194

This annex lists the patent family members relating to the patent documents cited in the above-mentioned European search report.
The members are as contained in the European Patent Office EDP file on
The European Patent Office is in no way liable for these particulars which are merely given for the purpose of information.

28-02-2022

| Patent document cited in search report | | Publication date | Patent family member(s) | | | Publication date |
|---|---|---|---|---|---|---|
| EP 1145707 | A1 | 17-10-2001 | AU | 2319400 | A | 18-08-2000 |
| | | | EP | 1145707 | A1 | 17-10-2001 |
| | | | KR | 20010101715 | A | 14-11-2001 |
| | | | WO | 0044341 | A1 | 03-08-2000 |
| US 2009176676 | A1 | 09-07-2009 | AU | 2005314016 | A1 | 15-06-2006 |
| | | | BR | PI0518973 | A2 | 16-12-2008 |
| | | | CA | 2588549 | A1 | 15-06-2006 |
| | | | CN | 101076373 | A | 21-11-2007 |
| | | | EP | 1819401 | A2 | 22-08-2007 |
| | | | JP | 2008521936 | A | 26-06-2008 |
| | | | US | 2006127345 | A1 | 15-06-2006 |
| | | | US | 2009176676 | A1 | 09-07-2009 |
| | | | WO | 2006063104 | A2 | 15-06-2006 |
| US 2006147390 | A1 | 06-07-2006 | DE | 10333710 | A1 | 24-02-2005 |
| | | | EP | 1656104 | A1 | 17-05-2006 |
| | | | EP | 1670429 | A1 | 21-06-2006 |
| | | | US | 2006147390 | A1 | 06-07-2006 |
| | | | WO | 2005009403 | A1 | 03-02-2005 |
| | | | WO | 2005009404 | A1 | 03-02-2005 |
| US 6599472 | B1 | 29-07-2003 | CA | 2360964 | A1 | 03-05-2002 |
| | | | US | 6599472 | B1 | 29-07-2003 |
| JP H1045543 | A | 17-02-1998 | JP | 3650677 | B2 | 25-05-2005 |
| | | | JP | H1045543 | A | 17-02-1998 |
| US 2009092568 | A1 | 09-04-2009 | NONE | | | |

EPO FORM P0459

For more details about this annex : see Official Journal of the European Patent Office, No. 12/82

14

**EP 3 981 383 A1**

**REFERENCES CITED IN THE DESCRIPTION**

**Non-patent literature cited in the description**

- Arctander, Perfume and Flavour Chemicals. *Aroma Chemicals,* 1969, vol. I,II